# FASCICULE DE BREVET EUROPEEN

(11) **EP 4 308 147 B1**
(45) Date de publication et mention de la délivrance du brevet: **23.07.2025**
(21) Numéro de dépôt: 22714187.6
(22) Date de dépôt: 14.03.2022
(51) Int. Cl.: A61K 38/17, A61K 9/08, A61K 9/06, A61K 47/36, A61K 38/42, A61K 35/62, A61P 17/10, A61P 17/02, A61P 1/02, A61P 1/00

(54) **GEL À BASE D'HÉMOGLOBINE D'ANNÉLIDES**
GEL AUF BASIS VON ANNELIDHÄMOGLOBIN
GEL BASED ON ANNELID HEMOGLOBIN

(30) Priorité: 15.03.2021 FR 2102533
(43) Date de publication de la demande: 24.01.2024
(73) Titulaire: Hemarina, 29600 Morlaix (FR)
(72) Inventeur: ZAL, Franck, 29600 MORLAIX (FR); LEIZE-ZAL, Elisabeth, 29600 MORLAIX (FR)
(74) Mandataire: Lavoix
(86) Numéro de dépôt international: PCT/EP2022/056448
(87) Numéro de publication internationale: WO 2022/194735

(56) Documents cités:
- EP-A1- 3 685 822
- WO-A1-2020/104440
- WO-A2-2009/007532
- CN-A- 108 354 951
- FR-A1- 3 076 713
- FR-A1- 3 100 035
- BATOOL FAREEHA ET AL: "A therapeutic oxygen carrier isolated from Arenicola marina decreased P. gingivalis induced inflammation and tissue destruction", vol. 10, no. 1, 1 December 2020 (2020-12-01), pages 14745 - 214745, XP055860593, Retrieved from the Internet <URL:https://www.nature.com/articles/s41598-020-71593-8.pdf> DOI: 10.1038/s41598-020-71593-8
- BATOOL FAREEHA ET AL: "Therapeutic Potential of Hemoglobin Derived from the Marine Worm Arenicola marina (M101): A Literature Review of a Breakthrough Innovation", MARINE DRUGS, vol. 19, no. 7, 1 July 2021 (2021-07-01), Basel, CH, pages 376, XP055860839, ISSN: 1660-3397, DOI: 10.3390/md19070376
- ÖZÇELIK HAYRIYE ET AL: "Characterization of a hyaluronic acid-based hydrogel containing an extracellular oxygen carrier (M101) for periodontitis treatment: An in vitro study", INTERNATIONAL JOURNAL OF PHARMACEUTICS, ELSEVIER, NL, vol. 605, 16 June 2021 (2021-06-16), XP086713642, ISSN: 0378-5173, [retrieved on 20210616], DOI: 10.1016/J.IJPHARM.2021.120810

## Description

La présente invention concerne une composition telle que définie en revendication 1.

Les maladies parodontales sont des maladies d'origine infectieuse (bactéries), qui touchent et détruisent les tissus de soutien des dents formant le parodonte. Le parodonte est constitué de quatre tissus : la gencive, l'os alvéolaire, le ligament alvéolo-dentaire et le cément. Lorsque la maladie parodontale se limite à la gencive, on parle de gingivite. Lorsqu'elle touche l'ensemble du parodonte, on parle de parodontite.

Les parodontites sont des maladies du système d'attache épithélio-conjonctif. Elles sont caractérisées par une perte d'attache, c'est-à-dire une dissociation de l'épithélium jonctionnel et des fibres conjonctives gingivales de la surface dentaire. Cette dissociation peut être pathologique, correspondant à la formation d'une poche parodontale, ou chirurgicale, correspondant à une plaie parodontale. La cicatrisation parodontale consiste donc en une réattache des tissus mous au niveau de la surface dentaire, à la « fermeture » en quelque sorte de la plaie parodontale.

Au sens large, la cicatrisation est la guérison d'une plaie, c'est-à-dire un processus dynamique intéressant l'ensemble des tissus de l'organisme et tendant à restaurer leur anatomie et leur fonction. La cicatrisation parodontale présente la particularité de mettre en jeu des tissus biologiquement distincts, de nature et de consistance différentes, contrairement au modèle de cicatrisation cutanée qui consiste en un affrontement et/ou un comblement de tissus mous histologiquement identiques.

La plaie cutanée est caractérisée par :
- deux tissus : l'épiderme et le derme ;
- un caillot dont le volume diffère en fonction de l'importance de la plaie ;
- des berges plus ou moins éloignées et soumises à des tensions.

Il est classique de distinguer la cicatrisation de première intention, résultant d'une parfaite coaptation des berges de la plaie, de la cicatrisation de deuxième intention, résultant de la migration des cellules épidermiques à partir de berges éloignées l'une de l'autre.

La cicatrisation parodontale apparaît plus complexe que la cicatrisation cutanée. On y retrouve les mêmes caractéristiques : compartiments tissulaires (épithélium et conjonctif), distance des berges, existence de contraintes, volume du caillot.

Néanmoins, ce modèle se différencie par (1) une plus grande diversité cellulaire associée à la participation des cellules osseuses et desmodontales, (2) des berges de la plaie de nature et de consistance différentes, et (3) un environnement bactérien spécifique. En effet, la plaque dentaire (i.e. accumulation de débris alimentaires et de bactéries) et le tartre (i.e. calcification de cette plaque dentaire) adhèrent à la surface de la dent située sous le rebord de la gencive, puis sont colonisés par des bactéries pathogènes. La stagnation des bactéries dans la plaque dentaire est à l'origine d'une réaction inflammatoire sur les gencives et l'os, induisant peu à peu leur destruction.

La cicatrisation des plaies, qu'elles soient cutanées ou parodontales, revêt donc un intérêt majeur pour restaurer l'intégrité de la barrière cutanée.

Le traitement actuel des parodontites consiste à effectuer un surfaçage radiculaire (au besoin sous anesthésie locale) pour éliminer la plaque dentaire et le tartre situés sous la gencive. L'objectif de ce traitement est de provoquer une ré-attache entre la gencive et les surfaces des racines précédemment exposées. Des chirurgies parodontales peuvent également être envisagées. Cependant, ces options restent relativement invasives.

Quant aux plaies cutanées, elles peuvent être plus ou moins sévères, et de différentes tailles.

Il existe donc un besoin pour un traitement efficace et simple des plaies, notamment des plaies cutanées ou parodontales, et qui favorise la cicatrisation.

Ces traitements doivent en outre être stériles pour ne pas contaminer d'avantage la peau et/ou le parodonte, mais aussi être stables. Enfin, ils doivent présenter une viscosité adéquate : ne pas être trop liquides pour rester un temps suffisant au niveau du site d'action (par exemple dans une poche parodontale ou sur la plaie cutanée), et ne pas être trop solides pour être résorbés relativement facilement.

La présente invention permet de répondre à ces attentes.

De façon surprenante, la Demanderesse a découvert que la formulation d'au moins une molécule choisie parmi une globine d'Annélides, un protomère de globine d'Annélides et une hémoglobine extracellulaire d'Annélides, dans un gel comprenant un polymère épaississant hydrophile d'origine naturelle ayant au moins une unité mannose et de l'acide hyaluronique ou son sel, permet d'obtenir une composition stable et efficace notamment dans la cicatrisation, notamment de la peau ou des plaies parodontales. En outre, une telle composition permet d'administrer la molécule *in situ* de manière fiable, et avec une durée d'action satisfaisante (i.e. au moins quelques heures, de préférence au moins 5 heures, de préférence au moins 10 heures, de préférence au moins 12 heures). Enfin, une telle composition est typiquement stérile, et présente la viscosité requise. Enfin, une telle composition est biocompatible, biodégradable et résorbable.

La présente invention a pour objet une composition (« composition selon l'invention ») telle que définie en revendication 1.

L'invention se rapporte également à l'utilisation d'une composition selon l'invention comme médicament.

De préférence, l'invention se rapporte à l'utilisation d'une composition selon l'invention pour prévenir et/ou traiter une maladie parodontale, et/ou pour traiter au moins une poche parodontale, et/ou pour favoriser la cicatrisation de la peau et/ou du parodonte et/ou pour traiter (i.e. favoriser et/ou augmenter) la cicatrisation osseuse.

De préférence, l'invention se rapporte à l'utilisation d'une composition selon l'invention pour prévenir et/ou traiter une maladie dermatologique, de préférence l'acné.

De préférence, la composition selon l'invention présente des propriétés rhéofluidisantes.

Ces propriétés peuvent être évaluées par le protocole suivant : on utilise un rhéomètre (Thermo Scientific Haake Mars, système de rhéomètre avancé modulaire) à géométrie de plaques parallèles (diamètre 40 mm, écart 1 mm). Une quantité appropriée d'échantillon est chargée sur une plaque peltier équipée d'un système à température contrôlée pour une thermorégulation efficace et précise (±0,001°C). Les courbes de débit de viscosité sont mesurées par une méthode de balayage à vitesse constante (0,1 à 10 000 Pa.s). La plage viscoélastique linéaire (Linear Viscoelastic Range ou LVER) de l'échantillon est déterminée par balayage dynamique d'amplitude et par les modules viscoélastiques (G' conservateur et G » » dissipatif) par test de balayage de fréquence. Dans ces tests, une excitation continue est appliquée à l'échantillon dans la LVER pour éviter la déstructuration de l'échantillon. La plage de test de balayage d'amplitude est comprise entre 0,01 et 100 % et avec une fréquence angulaire de 0,1 Hz à 20°C pour les deux essais. L'analyse de balayage de fréquence est effectuée sur l'échantillon dans le rhéomètre à 20°C dans la plage de fréquence angulaire entre 0,1 et 100 Hz en utilisant une tension constante de 1 %.

La composition selon l'invention présente des propriétés élastiques. En outre, G' reste supérieur à G" même à faible fréquence.

La composition selon l'invention comprend au moins une molécule choisie parmi une hémoglobine extracellulaire d'Annélides, ses globines et ses protomères de globine.

Cette molécule est un transporteur d'oxygène. Par « transporteur d'oxygène », on entend une molécule capable de transporter l'oxygène, de façon réversible, depuis l'environnement jusqu'aux cellules, tissus ou organes cibles.

L'hémoglobine extracellulaire d'Annélides est présente chez les trois classes d'Annélides: les Polychètes, les Oligochètes et les Achètes. On parle d'hémoglobine extracellulaire car elle est naturellement non contenue dans une cellule, et peut donc circuler librement dans le système sanguin sans modification chimique pour la stabiliser ou la rendre fonctionnelle. L'hémoglobine extracellulaire d'Annélides est un biopolymère géant de poids moléculaire compris entre 2000 et 4000 kDa, constitué d'environ 200 chaînes polypeptidiques comprises entre 4 et 12 types différents que l'on regroupe généralement en deux catégories.

La première catégorie, comptant 144 à 192 éléments, regroupe les chaînes polypeptidiques dites "fonctionnelles" qui portent un site actif de type hème, et sont capables de lier réversiblement l'oxygène ; ce sont des chaînes de type globine (huit types au total pour l'hémoglobine *d'Arenicola marina :* a1, a2, b1, b2, b3, c, d1 et d2), dont les masses sont comprises entre 15 et 18 kDa. Elles sont très similaires aux chaînes de type α et β de vertébrés.

La deuxième catégorie, comptant 36 à 42 éléments, regroupe les chaînes polypeptidiques dites de « structure » ou « linkers » possédant peu ou pas de site actif mais permettant l'assemblage des sous-unités appelées douzièmes ou protomères. On compte deux types de linkers, L1 et L2.

Chaque molécule d'hémoglobine est constituée de deux hexagones superposés que l'on a nommés bicouche hexagonale (hexagonal bilayer) et chaque hexagone est lui-même formé par l'assemblage de six sous-unités (dodécamère ou protomère) en forme de goutte d'eau. La molécule native est formée de douze de ces sous-unités (dodécamère ou protomère). Chaque sous-unité a une masse moléculaire d'environ 250 kDa, et constitue l'unité fonctionnelle de la molécule native.

De préférence, l'hémoglobine extracellulaire d'Annélides est choisie parmi les hémoglobines extracellulaires d'Annélides Polychètes et les hémoglobines extracellulaires d'Annélides Oligochètes. De préférence, l'hémoglobine extracellulaire d'Annélides est choisie parmi les hémoglobines extracellulaires de la famille des *Lumbricidae,* les hémoglobines extracellulaires de la famille des *Arenicolidae* et les hémoglobines extracellulaires de la famille des *Nereididae.* Encore plus préférentiellement, l'hémoglobine extracellulaire d'Annélides est choisie parmi l'hémoglobine extracellulaire de *Lumbricus terrestris,* l'hémoglobine extracellulaire *d'Arenicola sp* et l'hémoglobine extracellulaire de *Nereis sp.* Plus préférentiellement selon l'invention, l'hémoglobine extracellulaire *d'Arenicola marina* ou de *Nereis virens,* plus préférentiellement l'hémoglobine extracellulaire *d'Arenicola marina.* L'arénicole *Arenicola marina* est un ver annélide polychète vivant essentiellement dans le sable.

Selon l'invention, le protomère de globine de l'hémoglobine extracellulaire d'Annélides constitue l'unité fonctionnelle de l'hémoglobine native, comme indiqué ci-dessus.

Enfin, la chaîne de globine de l'hémoglobine extracellulaire d'Annélides peut notamment être choisie parmi les chaînes de globine de type Ax et/ou Bx d'hémoglobine extracellulaire d'Annélides.

L'hémoglobine extracellulaire d'Annélides, ses protomères de globine et/ou ses globines ne nécessitent pas de cofacteur pour fonctionner, contrairement à l'hémoglobine de mammifère, notamment humaine. Enfin, l'hémoglobine extracellulaire d'Annélides, ses protomères de globine et/ou ses globines ne possédant pas de typage sanguin, ils permettent d'éviter tout problème de réaction immunologique ou allergique. L'hémoglobine extracellulaire d'Annélides, ses protomères de globine et/ou ses globines présentent une activité superoxide dismutase (SOD) intrinsèque. Par conséquent, cette activité antioxydante intrinsèque ne nécessite aucun antioxydant pour fonctionner, contrairement à l'utilisation d'une hémoglobine de mammifères pour laquelle les molécules antioxydantes sont contenues à l'intérieur du globule rouge et ne sont pas liées à l'hémoglobine.

L'hémoglobine extracellulaire d'Annélides, ses protomères de globine et/ou ses globines peuvent être natifs ou recombinants.

De préférence, l'hémoglobine extracellulaire est celle *d'Arenicola marina* ou celle de *Nereis virens,* plus préférentiellement l'hémoglobine extracellulaire *d'Arenicola marina.*

De préférence, la molécule est présente dans la composition selon l'invention en une teneur comprise entre 0,01% et 10% en poids par rapport au poids total de composition, de préférence entre 0,05% et 5% en poids, de préférence entre 0,06% et 2% en poids, de préférence entre 0,07% et 1% en poids, de préférence entre 0,08% et 0,5% en poids, de préférence entre 0,09% et 0,3% en poids.

De préférence, la molécule choisie parmi une globine d'Annélides, un protomère de globine d'Annélides et une hémoglobine extracellulaire d'Annélides selon l'invention, est formulée dans une solution tampon. La solution obtenue (i.e. solution tampon comprenant la molécule) peut être lyophilisée, afin d'obtenir une poudre. De préférence, la solution obtenue (i.e. solution tampon comprenant la molécule) est utilisée telle quelle (forme liquide), sous forme non lyophilisée.

Typiquement, la solution tampon comprenant la molécule, lyophilisée ou non lyophilisée (et dans ce cas liquide), est introduite dans le mélange comprenant le polymère épaississant hydrophile et l'acide hyaluronique pour obtenir la composition selon l'invention.

La solution tampon crée un environnement salin adéquat pour l'hémoglobine, ses protomères et ses globines, et permet ainsi le maintien de la structure quaternaire, et donc de la fonctionnalité de cette molécule. La solution tampon est de préférence une solution aqueuse comprenant des sels, de préférence des ions chlorure, sodium, calcium, magnésium et potassium, et son pH est compris entre 5 et 9, de préférence entre 5.5 et 8.5, de préférence entre 6.5 et 7.6. Sa formulation est similaire à celle d'un liquide physiologiquement injectable. De préférence, la solution tampon comprend également un antioxydant, tel que l'acide ascorbique. Dans ces conditions, l'hémoglobine extracellulaire d'Annélides, ses protomères de globine et ses globines restent fonctionnels.

Dans la présente description, le pH s'entend à température ambiante (25°C), sauf mention contraire. De préférence, la solution tampon est une solution aqueuse comprenant du chlorure de sodium, du chlorure de calcium, du chlorure de magnésium, du chlorure de potassium, ainsi que du sodium gluconate et du sodium acétate, et a un pH compris entre 6.5 et 7.6, de préférence égal à 7,1 ± 0,5, de préférence d'environ 7.35. Plus préférentiellement, la solution tampon est une solution aqueuse comprenant 90 mM de NaCl, 23 Mm de Na-gluconate, 2,5 mM de CaCl₂, 27 mM de Na-acétate, 1,5 mM de MgCl₂, 5 mM de KCI, et a un pH de 7,1 ± 0,5.

La composition selon la divulgation comprend également au moins un polymère épaississant hydrophile choisi parmi les polymères d'origine naturelle comprenant au moins une unité mannose. Ces polymères d'origine naturelle peuvent être modifiés, par exemple par l'ajout d'un ou plusieurs groupements hydroxypropyl, par l'ajout de groupements de sels de methylcarboxylate tel le méthylcarboxylate de sodium, ou encore par l'ajout de groupes triméthyl ammonium.

Selon la divulgation, le polymère épaississant hydrophile est polymère d'origine naturelle non modifié comprenant au moins une unité mannose.

Selon la divulgation, le polymère épaississant hydrophile est choisi parmi :
- la gomme de xanthane et les dérivés du xanthane ;
- les glucomannanes et leurs dérivés, tels que la gomme de konjac ;
- les galactomannanes et leurs dérivés, tels que la gomme de caroube, la gomme de fenugrec, la gomme tara, la gomme de guar ou les dérivés de gomme de guar tels que l'hydroxypropylguar, l'hydroxypropylguar modifié par des groupements methylcarboxylate de sodium (tel que le produit vendu sous le nom Jaguar XC97-1 par Rhodia) ou le chlorure de guar hydroxypropyl triméthyl ammonium;
- et leurs mélanges.

Selon la divulgation, le polymère épaississant hydrophile est choisi parmi :
- la gomme de xanthane;
- la gomme de konjac ;
- la gomme de caroube, la gomme de fenugrec, la gomme tara, la gomme de guar;
- et leurs mélanges.

Le polymère épaississant hydrophile est choisi parmi la gomme de xanthane et les dérivés du xanthane.

La gomme de xanthane est un polyoside anionique de masse moléculaire élevée (environ 10⁶) produit par fermentation de glucides par *Xanthomonas campestris.* Elle se compose d'une chaîne principale constituée d'unités D-glucose liées par des ponts glycosidiques β(1->4) ; 1 unité anhydroglucose sur 2 porte une chaîne latérale triosidique constituée d'un résidu acide glucuronique compris entre 2 unités mannose. La plupart des unités terminales contiennent un groupement pyruvate et l'unité mannose adjacente à la chaîne principale peut être acétylée en C6.

Par exemple, la gomme de xanthane peut être vendue par Cargill sous la dénomination Satiaxane UCX 930 ou Satiaxane UCX 911.

De préférence, le polymère épaississant hydrophile est présent en quantité comprise entre 0,5% et 5% en poids par rapport au poids total de composition, de préférence entre 0,8% et 4% en poids, de préférence entre 1% et 3% en poids, de préférence entre 1,5% et 2,5% en poids.

La composition selon l'invention comprend également de l'acide hyaluronique ou l'un de ses sels.

L'acide hyaluronique est un polymère de disaccharide, en particulier un glycosaminoglycane, formé d'acide D-glucuronique et de N-acétyl-D-glucosamine.

Il est présent naturellement dans de nombreux tissus, en grande partie dans la peau, en particulier dans l'épiderme, ainsi que dans les tissus conjonctifs et représente l'un des principaux constituants de la matrice extracellulaire. La longueur de la molécule varie selon les tissus, l'espèce et l'état du tissu.

L'acide hyaluronique peut être obtenu par extraction tissulaire à partir de tissus animaux ou par fermentation bactérienne, notamment avec *Streptococcus equi* ou *Bacillus subtilis.*

De préférence, l'acide hyaluronique selon l'invention est obtenu par fermentation bactérienne, notamment avec *Streptococcus equi* ou *Bacillus subtilis,* plus particulièrement avec *Streptococcus equi.*

De préférence, la composition selon l'invention comprend un sel d'acide hyaluronique, appelé hyaluronate. De préférence la composition selon l'invention comprend un sel de sodium (hyaluronate de sodium). De préférence, l'acide hyaluronique ou l'un de ses sels est non sulfaté.

De préférence, la composition selon l'invention comprend de l'acide hyaluronique ou l'un de ses sels à une teneur d'au moins 0,1% en poids, de préférence au moins 0,2% en poids, de préférence au moins 0,3% en poids, de préférence au moins 0,4% en poids, de préférence au moins 0,5% en poids par rapport au poids total de la composition.

De préférence, l'acide hyaluronique ou l'un de ses sels est présent en quantité comprise entre 0,3% et 5% en poids par rapport au poids total de composition, de préférence entre 0,5% et 3% en poids, de préférence entre 0,8% et 2% en poids.

L'acide hyaluronique ou l'un de ses sels peut être un acide hyaluronique ou un hyaluronate de bas poids moléculaire, un acide hyaluronique ou un hyaluronate de haut poids moléculaire, ou un mélange des deux.

L'acide hyaluronique ou un hyaluronate de haut poids moléculaire peut avoir un poids moléculaire allant de 5 à 5000 kDa, notamment de 6 à 4800 kDa, en particulier de 8 à 4500 kDa (4,5 MDa). De préférence, l'acide hyaluronique ou un hyaluronate de haut poids moléculaire a un poids moléculaire allant de 1400 à 4000 kDa, de préférence de 1500 à 3500 kDa, de préférence de 1500 à 3400 kDa.

L'acide hyaluronique ou un hyaluronate de bas poids moléculaire peut avoir un poids moléculaire allant de 10 à 1000 kDa, notamment de 10 à 600 kDa.

Le poids moléculaire peut être mesuré par la classique méthode HPLC élution/exclusion. Selon un mode de réalisation particulier, la composition comprend comme acide hyaluronique ou l'un de ses sels uniquement de l'acide hyaluronique ou un hyaluronate de haut poids moléculaire. Ceci signifie notamment que le rapport pondéral en acide hyaluronique (ou hyaluronate) de bas poids moléculaire par rapport à l'acide hyaluronique (ou hyaluronate) de haut poids moléculaire est inférieur ou égal à 0,1%, en particulier inférieur à 0,01%, voire est de 0.

En particulier, la composition comprend une teneur en acide hyaluronique (ou hyaluronate) de haut poids moléculaire comprise entre 0,3% et 5% en poids par rapport au poids total de composition, de préférence entre 0,5% et 3% en poids, de préférence entre 0,8% et 2% en poids.

De préférence, l'acide hyaluronique ou l'un de ses sels présente une viscosité intrinsèque à 25°C allant de 1 à 4 m³/kg, de préférence de 1,4 à 3,8 m³/kg, de préférence de 1,7 à 3,4 m³/kg, de préférence de 2 à 3,4 m³/kg, de préférence de 2,5 à 3,4 m³/kg. Le calcul de viscosité intrinsèque est un paramètre bien connu de l'homme du métier, et peut être effectué comme indiqué dans la Pharmacopée Européenne (Pharmacopée Européenne 9.0, Monographies S, Sodium (hyaluronate de), pages 3834-3835).

De préférence, la composition selon l'invention comprend, dans un milieu aqueux physiologiquement acceptable :
- une hémoglobine extracellulaire d'*Arenicola marina* ou celle de *Nereis virens,* de préférence en une teneur comprise entre 0,05% et 0,5% en poids par rapport au poids total de composition, de préférence entre 0,08% et 0,4% en poids, de préférence entre 0,09% et 0,3% en poids,
- de la gomme de xanthane, de préférence en une teneur comprise entre 0,5% et 5% en poids par rapport au poids total de composition, de préférence entre 1% et 3% en poids, de préférence entre 1,5% et 2,5% en poids, et
- du hyaluronate de sodium, de préférence de haut poids moléculaire, de préférence en une teneur comprise entre 0,4% et 5% en poids par rapport au poids total de composition, de préférence entre 0,5% et 4% en poids, de préférence entre 0,8% et 2% en poids.

De préférence, la composition selon l'invention comprend, dans un milieu aqueux physiologiquement acceptable :
- une hémoglobine extracellulaire d'*Arenicola marina,* en une teneur comprise entre 0,09% et 0,3% en poids,
- de la gomme de xanthane, en une teneur comprise entre 1,5% et 2,5% en poids, et
- du hyaluronate de sodium, de préférence de viscosité intrinsèque à 25°C allant de 1 à 4 m³/kg, de préférence de 1,4 à 3,8 m³/kg, de préférence de 1,7 à 3,4 m³/kg, de préférence de 2 à 3,4 m³/kg, de préférence de 2,5 à 3,4 m³/kg, en une teneur comprise entre 0,8% et 2% en poids.

La composition selon l'invention comprend également un milieu aqueux physiologiquement acceptable. Par « physiologiquement acceptable », on entend que le milieu est compatible avec une application sur la peau et/ou dans la poche parodontale. De préférence, ledit milieu est stérile.

Le milieu comprend typiquement de l'eau. De préférence, la quantité d'eau est d'au moins 80% en poids, de préférence au moins 90% en poids, de préférence au moins 95% en poids par rapport au poids total de composition.

De préférence, la composition selon l'invention est un gel.

De préférence, la composition selon l'invention est administrée par injection ou par instillation *in situ* dans la zone à traiter. De préférence, en particulier dans le cas de pathologies parodontales, la composition selon l'invention est administrée par voie locale, par injection ou par instillation *in situ* dans le creux de la poche parodontale ou sur la plaie. De préférence, la composition selon l'invention est administrée par instillation directement dans la zone à traire, typiquement dans le creux de la poche parodontale ou sur la plaie à traiter. Elle peut également être administrée au niveau de l'os du parodonte, directement sur l'os ou bien de manière adjacente.

De préférence, la composition selon l'invention est utilisée en thérapie, de préférence pour prévenir et/ou traiter une maladie parodontale, et/ou pour traiter au moins une poche parodontale, et/ou pour favoriser la cicatrisation de la peau et/ou du parodonte. De préférence, la composition selon l'invention est utilisée pour prévenir et/ou traiter une maladie parodontale, en favorisant la ré-attache entre la gencive et les surfaces des racines dentaires, mais aussi en favorisant la cicatrisation du tissu osseux.

De préférence, la composition selon l'invention est utilisée pour traiter (i.e. favoriser et/ou augmenter) la cicatrisation osseuse.

De préférence, la composition selon l'invention est utilisée comme anti-inflammatoire, notamment pour traiter l'inflammation induite par l'hypoxie, et/ou pour inhiber la dégradation des tissus induite par *P.gingivalis.*

Comme cela est explicité en exemples, l'action anti-inflammatoire de la composition selon l'invention permet de prévenir et/ou traiter une maladie parodontale ou une poche parodontale : en inhibant l'inflammation induite par l'hypoxie dans ce type de pathologie, la composition selon l'invention permet de freiner l'évolution de la parodontite.

Par ailleurs, la composition selon l'invention inhibe la dégradation des tissus induite par *P.gingivalis,* et favorise ainsi la cicatrisation.

De préférence, la composition selon l'invention est utilisée pour prévenir et/ou traiter une maladie dermatologique, de préférence une maladie dermatologique inflammatoire, de préférence l'acné. Par « maladie dermatologique inflammatoire », on entend tout désordre cutané accompagné d'une composante inflammatoire. Le terme inclut notamment la rosacée, l'acné, l'eczéma, l'eczéma des mains, l'urticaire, l'érythème facial et pudique, le prurit, la dermatite atopique et le psoriasis sous toutes ses formes tel que cutané, muqueux ou unguéal, ou le rhumatisme psoriasique.

En effet, la composition selon l'invention peut inhiber l'inflammation causée par les bactéries impliquées dans ces pathologies ; les maladies dermatologiques telles que l'acné impliquent une composante bactérienne et inflammatoire. En tant que membre de la microflore humaine résidente, la bactérie anaérobie à Gram positif *Propionibacterium acnes (P.acnes,* appelée aujourd'hui *Cutibacterium acnes*) se trouve principalement dans la glande sébacée de la peau. *P.acnes* a une densité cutanée estimée de 10² à 10⁵⁻⁶ cm⁻² ; c'est un pathogène opportuniste bien connu. Parfois, cette bactérie, qui vit normalement à la surface de la peau, provoque une inflammation (mais pas une infection) des follicules pileux. Si l'inflammation se développe près de la surface de la peau, des taches rouges ou jaunes (pustules) peuvent se former. Des lésions enflammées plus profondes (nodules et kystes) peuvent se former si l'infection est plus proche de la racine du cheveu. Dans l'acné très sévère, les kystes peuvent se rassembler pour former des lésions enflammées encore plus grandes et plus profondes (acné conglobata), mais cela est rare. *P.acnes* et les lésions peuvent également être secondairement infectées par *Staphyloccocus aureus.*

De préférence, l'acné est choisie parmi les acnés vulgaires, comédoniennes, polymorphes, les acnés causées par une rosacée, les acnés nodulokystiques, conglobata et les acnés séniles.

L'invention se rapporte également à un dispositif comprenant :
- une seringue, et
- une composition selon l'invention.

La seringue contient la composition selon l'invention.

Dans le dispositif selon l'invention, la seringue peut être reliée à une aiguille creuse, de préférence à une aiguille creuse pourvue d'un trou latéral. Typiquement, une telle aiguille peut être présente ou non lors de l'administration de la composition selon l'invention dans le creux d'une poche parodontale ; si elle n'est pas présente, dans ce cas, la composition sort directement de la seringue. De préférence, la composition selon l'invention est appliquée au niveau d'une poche parodontale ou de tout défaut cavitaire avec une aiguille creuse, de préférence une aiguille creuse pourvue d'un trou latéral, de préférence avec extrémité arrondie. De préférence, la composition selon l'invention est appliquée sur la peau (application cutanée) directement en sortant de la seringue.

L'invention est illustrée par les exemples suivants.

### Exemple 1 : Préparation d'une composition selon l'invention

Dans des conditions stériles, la composition suivante est préparée :

**[Table 1]**

| **Ingrédient** | **Quantité** |
|---|---|
| Hémoglobine extracellulaire *d'Arenicola marina* dans une solution tampon (Hemarina) | 1 g/l |
| Gomme de xanthane | 2 (% en poids par rapport au poids total de composition) |
| Hyaluronate de sodium (Sodium Hyaluronate d'HTL ou de Contipro) | 1 (% en poids par rapport au poids total de composition) |
| Tampon | Qsp 100 |
| | (% en poids par rapport au poids total de composition) |

On mélange tous les ingrédients dans un tampon jusqu'à obtenir un gel. La composition se présente sous forme de gel.

Une fois préparée, la composition est conditionnée dans des seringues de 1ml. Les seringues sont congelées.

La stabilité est ensuite évaluée.

Il ressort que la composition est stable pendant au moins 3 mois à 4°C, et que l'hémoglobine de la composition est également stable et fonctionnelle.

### Exemple 2 : Effet de la composition selon l'invention sur la cicatrisation

### 1. But de l'étude

Cette étude a pour objectif d'évaluer l'efficacité et la sécurité de produits cicatrisants, dont le gel selon l'invention, dans la cicatrisation de plaies chez le miniporc.

L'étude a duré 22 jours, et a été arrêtée suite à la cicatrisation de la majeure partie des plaies.

Elle porte sur 3 miniporcs provenant d'Ellegaard : M1, M2 et M3 (M1 et M2 sont des mâles et M3 une femelle).

Les animaux ont été hébergés selon des procédures standard.

### 2. Design de l'étude

### 2.1. Produits de traitement

Le design est explicité dans le tableau ci-dessous.

**[Table 2]**

| **Groupe** | **Dénomination** | **Description** |
|---|---|---|
| 1 | Pansement hydrocellulaire (Urgo Tul Border) | Pansement prêt à l'emploi |
| 2 | NaCl 0,9% | Solution injectable prête à l'emploi |
| 3 | Gel K-Y lubricating jelly (Reckitt Benckiser Healthcare) | Lubrifiant stérile mais non gras (à base d'oxyde de propylène et de gluconate de chlorhexidine, dans une matrice de mucilages végétaux, glycérine et eau) |
| 4 | Gel selon l'invention (Hemarina) | Composition selon l'invention conditionnée en seringue de 1 mL (tel que préparé dans l'exemple 1) |

### 2.2. Schéma de traitement

Les traitements ont été réalisés le jour de la création des plaies (J1) et à chaque changement de pansement soit : J1, J3, J5, J8, J10, J12, J15, J17 et J19.

La plaie a été nettoyée avant application du produit :
- Pansement hydrocellulaire : le pansement a été directement appliqué sur la plaie à la place du pansement Mepore^{®} (Mölnlycke, 6 x 7 cm) ;
- NaCl 0,9% : la partie absorbante du pansement Mepore^{®} a été imbibée avec du NaCl 0,9% avant l'application du pansement ;
- Gel K-Y lubricating jelly : le produit a été appliqué directement sur la plaie avant mise en place du pansement Mepore^{®} ;
- Gel selon l'invention : le produit a été appliqué directement sur la plaie avant mise en place du pansement Mepore^{®}.

### 2.3. Réalisation des plaies

Deux types de plaies de pleine épaisseur ont été réalisées: ronde au moyen d'un poinçon de biopsie, et carrée au moyen d'un scalpel. Huit (8) plaies ont été réalisées par animal (4 rondes / 20 mm de diamètre et 4 carrées / 20 mm de côté), permettant 4 traitements, chaque traitement était représenté par type de plaie).

### 2.3. Evaluation macroscopique des plaies

L'évaluation macroscopique des plaies a été effectuée avec les paramètres suivants : Une évaluation macroscopique a été réalisée à chaque changement de traitement / pansement (documentée par des photos et des échelles de notation).

### 3. Résultats

### 3.1. Commentaire général

Les retours sont positifs : le gel selon l'invention, disposé dans une seringue, est facile à utiliser et à appliquer, le produit restant bien en place dans la plaie pendant le traitement.

### 3.2. Evaluation macroscopique des plaies

Les observations macroscopiques étaient celles attendues pour ce type d'étude.

Les premiers signes d'épithélialisation sont apparus à partir de J5 pour le gel K-Y, le gel selon l'invention et le pansement hydrocellulaire, et à partir de J8 pour le NaCl.

Les réactions locales autour de la plaie (érythème, œdème et induration) ont été limitées et ont principalement concerné les premiers jours de l'étude ; elles s'expliquent par la présence d'un excès de produit appliqué (érythème pour gel K-Y ou gel selon l'invention). Il n'y a pas eu de réaction locale autour de la plaie avec le NaCl.

En ce qui concerne la cicatrisation globale de la plaie, on peut noter qu'à la fin de l'étude (J22) toutes les plaies traitées avec le gel selon l'invention étaient complètement cicatrisées, tandis qu'avec le gel KY et NaCl, une plaie n'était pas encore guérie, et qu'avec le pansement hydrocellulaire toutes les blessures n'étaient pas encore complètement guéries.

Il est également rapporté que le traitement avec le gel selon l'invention a causé beaucoup moins de croûtes que les autres traitements.

Par conséquent, le gel selon l'invention présente de très bonnes propriétés de cicatrisation.

### Exemple 3 : Effet de la composition selon l'invention sur les maladies parodontales

Objectifs: La parodontite est caractérisée par des poches parodontales profondes associées à une flore dysbiotique et à un microenvironnement hypoxique qui exacerbe l'inflammation et la dégradation des tissus.

L'hémoglobine extracellulaire d'*Arenicola marina* a un excellent potentiel de transport d'oxygène et une capacité antioxydante, et a récemment démontré des propriétés antiinflammatoires et antibactériennes.

Le but de cette étude est d'évaluer le rôle de cette hémoglobine dans la réduction de l'hypoxie et du stress oxydatif *in vitro* et *in vivo.*

Méthodes: Les cellules épithéliales orales en culture 2D et 3D ont été infectées par *P.gingivalis* (MOI = 100) ou exposées au chlorure de cobalt pour induire une hypoxie et un stress oxydatif, et traitées par l'hémoglobine (1g/L) pendant 24h.

L'hypoxie est évaluée par microscopie à fluorescence et quantification de l'expression des marqueurs clés de l'hypoxie (sous-unité α du facteur inductible par l'hypoxie (HIF-1α), transporteur de glucose-1 (Glut-1) et transporteur de glucose-3 (Glut-3)) par RT-qPCR et Elisa.

*In vivo,* une parodontite expérimentale a été induite par placement de ligature imbibées de *P.gingivalis* pendant 3 semaines, et les lésions ont été traitées par application du gel selon l'invention.

La guérison a été évaluée par analyse histomorphométrique et coloration TRAP après 2 semaines.

Résultats: En culture, le traitement avec le gel selon l'invention a réduit l'hypoxie et le stress oxydatif induits par *P.gingivalis* et le chlorure de cobalt. Après 24h de traitement, l'expression génique relative des marqueurs clés de l'hypoxie induite par *P.gingivalis* (HIF-1α, Glut-1, Glut-3) a été diminuée de 85%, 42% et 83% respectivement.

*In vivo,* les analyses histomorphométriques ont montré une réduction du score inflammatoire et une amélioration de l'attache clinique chez les souris traitées avec le gel selon l'invention par rapport au contrôle (p <0,05) et une activité ostéoclastique réduite. L'analyse immunohistochimique a également révélé une diminution de l'expression de HIF-1α dans les tissus mous chez le groupe traité par le gel de l'invention.

Conclusions: L'hémoglobine extracellulaire *d'Arenicola marina* est une molécule très prometteuse capable d'améliorer l'inflammation induite par l'hypoxie et la dégradation des tissus induite par *P.gingivalis.*

Par conséquent, elle présente un potentiel thérapeutique important dans la prise en charge de la parodontite.

### Exemple 4 : Essais comparatifs

Tous les pourcentages sont en poids par rapport au poids total de composition.

### 1/ Les compositions comparatives suivantes sont préparées par mélange des ingrédients :

Elles sont comparatives avec la formule selon l'invention de l'exemple 1.

**[Table 3]**

| **Composition** | **HA** | **XG** |
|---|---|---|
| **Ingrédient** | **Quantité** | **Quantité** |
| Hémoglobine extracellulaire *d'Arenicola marina* dans une solution tampon (Hemarina) | 1 g/l | 1 g/l |
| Gomme de xanthane | - | 3 (% en poids par rapport au poids total de composition) |
| Hyaluronate de sodium (Sodium Hyaluronate d'HTL, viscosité intrinsèque de 3.07 m³/kg) | 1 (% en poids par rapport au poids total de composition) | - |
| Tampon | Qsp 100 (% en poids par rapport au poids total de composition) | Qsp 100 (% en poids par rapport au poids total de composition) |

| | | |
|---|---|---|
| -La stabilité de la viscosité de la composition HA est évaluée à 37°C+/- 2°C à T0 et à T+12h. Les mesures de viscosité sont enregistrées au plateau newtonien à 37°C+/- 2°C pour une vitesse de cisaillement de 0,01 s⁻¹. Cette mesure est la mesure à T0. Après la mesure à T0, les gels sont placés au bain-marie à 37°C+/- 2°C pendant 12h. Les mesures de viscosité sont alors enregistrées au plateau newtonien à 37°C+/- 2°C pour une vitesse de cisaillement de 0,01 s⁻¹. Cette mesure est la mesure à T+12h. | | |

La composition HA, à 37°C+/- 2°C, ne présente pas une viscosité stable pendant la durée minimale nécessitée par l'usage.

-La vitesse de transfert du dioxygène entre deux compartiments séparés par des milieux de composition différente (i.e. eau faiblement minéralisée et saturée en O2, versus eau faiblement minéralisée et désaturée en O2) a été mesurée pour la composition XG. Les résultats montrent que cette composition XG présente une vitesse de transfert inférieure à la composition de l'exemple 1 (données non montrées).

-La composition de l'exemple 1 selon l'invention présente une viscosité adéquate, et une vitesse de transfert du dioxygène supérieure à la composition XG.

Ces résultats montrent que seule la composition selon l'invention permet d'obtenir une composition stable, présentant la viscosité adéquate et permettant une bonne diffusion du dioxygène.

## Revendications

1. Composition comprenant, dans un milieu aqueux physiologiquement acceptable :
- au moins une molécule choisie parmi une hémoglobine extracellulaire d'Annélides, ses globines et ses protomères de globine,
- au moins un polymère épaississant hydrophile choisi parmi la gomme de xanthane et les dérivés du xanthane, et
- de l'acide hyaluronique ou l'un de ses sels.

2. Composition selon la revendication 1, **caractérisée en ce que** la molécule est choisie parmi les hémoglobines extracellulaires de la famille des *Lumbricidae,* les hémoglobines extracellulaires de la famille des *Arenicolidae* et les hémoglobines extracellulaires de la famille des *Nereididae,* préférentiellement parmi l'hémoglobine extracellulaire de *Lumbricus terrestris,* l'hémoglobine extracellulaire d'*Arenicola sp* et l'hémoglobine extracellulaire de *Nereis sp,* plus préférentiellement parmi l'hémoglobine extracellulaire *d'Arenicola marina* et de *Nereis virens,* plus préférentiellement l'hémoglobine extracellulaire *d'Arenicola marina.*

3. Composition selon l'une des revendications précédentes, **caractérisée en ce que** l'acide hyaluronique ou l'un de ses sels est obtenu par fermentation bactérienne, notamment avec *Streptococcus equi* ou *Bacillus subtilis,* plus particulièrement avec *Streptococcus equi.*

4. Composition selon l'une des revendications précédentes, **caractérisée en ce que** l'acide hyaluronique ou l'un de ses sels est le hyaluronate de sodium.

5. Composition selon l'une des revendications précédentes, **caractérisée en ce que** l'acide hyaluronique ou l'un de ses sels a un poids moléculaire allant de 1400 à 4000 kDa, de préférence de 1500 à 3500 kDa, de préférence de 1500 à 3400 kDa.

6. Composition selon l'une des revendications précédentes, **caractérisée en ce que** l'acide hyaluronique ou l'un de ses sels présente une viscosité intrinsèque à 25°C allant de 1 à 4 m³/kg, de préférence de 1,4 à 3,8 m³/kg, de préférence de 1,7 à 3,4 m³/kg, de préférence de 2 à 3,4 m³/kg, de préférence de 2,5 à 3,4 m³/kg.

7. Composition selon l'une des revendications précédentes, **caractérisée en ce que** la molécule est présente en quantité comprise entre 0,01% et 10% en poids par rapport au poids total de composition, de préférence entre 0,05% et 5% en poids, de préférence entre 0,06% et 2% en poids, de préférence entre 0,07% et 1% en poids, de préférence entre 0,08% et 0,5% en poids, de préférence entre 0,09% et 0,3% en poids.

8. Composition selon l'une des revendications précédentes, **caractérisée en ce que** le polymère épaississant hydrophile est présent en quantité comprise entre 0,5% et 5% en poids par rapport au poids total de composition, de préférence entre 0,8% et 4% en poids, de préférence entre 1% et 3% en poids ; et/ou l'acide hyaluronique ou l'un de ses sels est présent en quantité comprise entre 0,3% et 5% en poids par rapport au poids total de composition, de préférence entre 0,5% et 3% en poids, de préférence entre 0,8% et 2% en poids.

9. Composition selon l'une des revendications précédentes, **caractérisée en ce qu'**elle comprend :
- une hémoglobine extracellulaire *d'Arenicola marina* ou celle de *Nereis virens,* de préférence en une teneur comprise entre 0,05% et 0,5% en poids par rapport au poids total de composition, de préférence entre 0,08% et 0,4% en poids, de préférence entre 0,09% et 0,3% en poids,
- de la gomme de xanthane, de préférence en une teneur comprise entre 0,5% et 5% en poids par rapport au poids total de composition, de préférence entre 1% et 3% en poids, de préférence entre 1,5% et 2,5% en poids, et
- du hyaluronate de sodium, de préférence de haut poids moléculaire, de préférence en une teneur comprise entre 0,4% et 5% en poids par rapport au poids total de composition, de préférence entre 0,5% et 4% en poids, de préférence entre 0,8% et 2% en poids.

10. Composition selon l'une des revendications précédentes, **caractérisée en ce qu'**elle comprend :
- une hémoglobine extracellulaire *d'Arenicola marina,* en une teneur comprise entre 0,09% et 0,3% en poids,
- de la gomme de xanthane, en une teneur comprise entre 1,5% et 2,5% en poids, et
- du hyaluronate de sodium, en une teneur comprise entre 0,8% et 2% en poids.

11. Dispositif comprenant :
- une seringue, et
- une composition selon l'une des revendications précédentes.

12. Composition selon l'une des revendications 1 à 10, pour son utilisation comme médicament.

13. Composition selon l'une des revendications 1 à 10, pour son utilisation pour prévenir et/ou traiter une maladie parodontale, et/ou pour traiter au moins une poche parodontale, et/ou pour favoriser la cicatrisation de la peau et/ou du parodonte et/ou pour traiter la cicatrisation osseuse, et/ou pour prévenir et/ou traiter une maladie dermatologique, de préférence l'acné.

## Patentansprüche

1. Zusammensetzung, umfassend in einem physiologisch annehmbaren wässrigen Medium:
- mindestens ein Molekül, das ausgewählt ist aus extrazellulärem Hämoglobin von Anneliden, seinen Globinen und seinen Globinprotomeren,
- mindestens ein hydrophiles verdickendes Polymer, ausgewählt aus Xanthangummi und Xanthanderivaten, und
- Hyaluronsäure oder eines ihrer Salze.

2. Zusammensetzung nach Anspruch 1, **dadurch gekennzeichnet, dass** das Molekül ausgewählt ist aus extrazellulärem Hämoglobin der Familie *Lumbricidae,* extrazellulärem Hämoglobin der Familie *Arenicolidae* und extrazellulärem Hämoglobin der Familie *Nereididae,* vorzugsweise aus dem extrazellulären Hämoglobin von *Lumbricus terrestris,* dem extrazellulären Hämoglobin von *Arenicola sp* und dem extrazellulären Hämoglobin von *Nereis sp,* bevorzugter aus dem extrazellulären Hämoglobin von *Arenicola marina* und *Nereis virens,* bevorzugter aus dem extrazellulären Hämoglobin von *Arenicola marina.*

3. Zusammensetzung nach einem der vorherigen Ansprüche, **dadurch gekennzeichnet, dass** die Hyaluronsäure oder eines ihrer Salze durch bakterielle Fermentation, insbesondere mit *Streptococcus equi* oder *Bacillus subtilis,* insbesondere mit *Streptococcus equi,* erlangt wird.

4. Zusammensetzung nach einem der vorherigen Ansprüche, **dadurch gekennzeichnet, dass** die Hyaluronsäure oder eines ihrer Salze Natriumhyaluronat ist.

5. Zusammensetzung nach einem der vorherigen Ansprüche, **dadurch gekennzeichnet, dass** die Hyaluronsäure oder eines ihrer Salze ein Molekulargewicht von 1400 bis 4000 kDa, vorzugsweise von 1500 bis 3500 kDa, vorzugsweise von 1500 bis 3400 kDa, aufweist.

6. Zusammensetzung nach einem der vorherigen Ansprüche, **dadurch gekennzeichnet, dass** die Hyaluronsäure oder eines ihrer Salze eine intrinsische Viskosität bei 25 °C von 1 bis 4 m³/kg, vorzugsweise von 1,4 bis 3,8 m³/kg, vorzugsweise von 1,7 bis 3,4 m³/kg, vorzugsweise von 2 bis 3,4 m³/kg, vorzugsweise von 2,5 bis 3,4 m³/kg, aufweist.

7. Zusammensetzung nach einem der vorherigen Ansprüche, **dadurch gekennzeichnet, dass** das Molekül in einer Menge zwischen 0,01 Gewichts-% und 10 Gewichts-%, bezogen auf das Gesamtgewicht der Zusammensetzung, vorzugsweise zwischen 0,05 Gewichts-% und 5 Gewichts-%, vorzugsweise zwischen 0,06 Gewichts-% und 2 Gewichts-%, vorzugsweise zwischen 0,07 und 1 Gewichts-%, vorzugsweise zwischen 0,08 Gewichts-% und 0,5 Gewichts-%, vorzugsweise zwischen 0,09 Gewichts-% und 0,3 Gewichts-%, vorhanden ist.

8. Zusammensetzung nach einem der vorherigen Ansprüche, **dadurch gekennzeichnet, dass** das hydrophile verdickende Polymer in einer Menge zwischen 0,5 Gewichts-% und 5 Gewichts-%, bezogen auf das Gesamtgewicht der Zusammensetzung, vorzugsweise zwischen 0,8 Gewichts-% und 4 Gewichts-%,vorzugsweise zwischen 1 Gewichts-% und 3 Gewichts-%, vorhanden ist; und/oderHyaluronsäure oder eines ihrer Salze in einer Menge zwischen 0,3 Gewichts-% und 5 Gewichts-%, bezogen auf das Gesamtgewicht der Zusammensetzung, vorzugsweise zwischen 0,5 Gewichts-% und 3 Gewichts-%, vorzugsweise zwischen 0,8 Gewichts-% und 2 Gewichts-%, vorhanden ist.

9. Zusammensetzung nach einem der vorherigen Ansprüche, **dadurch gekennzeichnet, dass** sie Folgendes umfasst:
- ein extrazelluläres Hämoglobin von *Arenicola marina* oder das von *Nereis virens,* vorzugsweise in einem Gehalt zwischen 0,05 Gewichts-% und 0,5 Gewichts-%, bezogen auf das Gesamtgewicht der Zusammensetzung, vorzugsweise zwischen 0,08 Gewichts-% und 0,4 Gewichts-%, vorzugsweise zwischen 0,09 Gewichts-% und 0,3 Gewichts-%,
- Xanthangummi, vorzugsweise in einem Gehalt zwischen 0,5 Gewichts-% und 5 Gewichts-%, bezogen auf das Gesamtgewicht der Zusammensetzung, vorzugsweise zwischen 1 Gewichts-% und 3 Gewichts-%, vorzugsweise zwischen 1,5 Gewichts-% und 2,5 Gewichts-%, und
- Natriumhyaluronat, vorzugsweise mit hohem Molekulargewicht, vorzugsweise in einem Gehalt zwischen 0,4 Gewichts-% und 5 Gewichts-%, bezogen auf das Gesamtgewicht der Zusammensetzung, vorzugsweise zwischen 0,5 Gewichts-% und 4 Gewichts-%, vorzugsweise zwischen 0,8 Gewichts-% und 2 Gewichts-%.

10. Zusammensetzung nach einem der vorherigen Ansprüche, **dadurch gekennzeichnet, dass** sie Folgendes umfasst:
- extrazelluläres Hämoglobin von *Arenicola marina,* mit einem Gehalt zwischen 0,09 Gewichts-% und 0,3 Gewichts-%,
- Xanthangummi, mit einem Gehalt zwischen 1,5 Gewichts-% und 2,5 Gewichts-%, und
- Natriumhyaluronat mit einem Gehalt zwischen 0,8 Gewichts-% und 2 Gewichts-%.

11. Vorrichtung, umfassend:
- eine Spritze, und
- eine Zusammensetzung nach einem der vorherigen Ansprüche.

12. Zusammensetzung nach einem der Ansprüche 1 bis 10 zur Verwendung als Medikament.

13. Zusammensetzung nach einem der Ansprüche 1 bis 10 zur Verwendung zur Prävention und/oder Behandlung einer Parodontalerkrankung und/oder zur Behandlung mindestens einer Parodontaltasche und/oder zur Förderung der Heilung der Haut und/oder des Parodonts und/oder zur Behandlung der Knochenheilung und/oder zur Vorbeugung und/oder Behandlung einer dermatologischen Erkrankung, vorzugsweise Akne.

## Claims

1. Composition comprising, in a physiologically acceptable aqueous medium:
- at least one molecule chosen from an Annelid extracellular hemoglobin, its globins and its globin protomers;
- at least one hydrophilic thickening polymer chosen from xanthan gum and xanthan derivatives; and
- hyaluronic acid or one of its salts.

2. Composition according to claim 1, **characterized in that** the molecule is chosen from among the extracellular hemoglobins of the *Lumbricidae* family, the extracellular hemoglobins of the *Arenicolidae* family, and the extracellular hemoglobins of the *Nereididae* family, preferably from among the extracellular hemoglobin of *Lumbricus terrestris,* the extracellular hemoglobin of *Arenicola sp* and the extracellular hemoglobin of *Nereis sp,* more preferably from among the extracellular hemoglobin of *Arenicola marina* and *Nereis virens,* more preferably the extracellular hemoglobin of *Arenicola marina.*

3. Composition according to one of the preceding claims, **characterized in that** the hyaluronic acid or one of its salts is obtained by bacterial fermentation in particular with *Streptococcus equi* or *Bacillus subtilis,* more particularly with *Streptococcus equi.*

4. Composition according to one of the preceding claims, **characterized in that** the hyaluronic acid or one of its salts is sodium hyaluronate.

5. Composition according to one of the preceding claims, **characterized in that** the hyaluronic acid or one of its salts has a molecular weight ranging from 1400 to 4000 kDa, preferably from 1500 to 3500 kDa, preferably from 1500 to 3400 kDa.

6. Composition according to one of the preceding claims, **characterized in that** the hyaluronic acid or one of its salts has intrinsic viscosity at 25°C ranging from 1 to 4 m³/kg, preferably from 1.4 to 3.8 m³/kg, preferably from 1.7 to 3.4 m³/kg, preferably from 2 to 3.4 m³/kg, preferably from 2.5 to 3.4 m³/kg.

7. Composition according to one of the preceding claims, **characterized in that** the molecule is present in an amount of between 0.01 % and 10 % by weight relative to the total weight of the composition, preferably between 0.05 % and 5 % by weight, preferably between 0.06 % and 2 % by weight, preferably between 0.07 % and 1 % by weight, preferably between 0.08 % and 0.5 % by weight, preferably between 0.09 % and 0.3 % by weight.

8. Composition according to one of the preceding claims, **characterized in that** the hydrophilic thickening polymer is present in an amount of between 0.5 % and 5 % by weight relative to the total weight of the composition, preferably between 0.8 % and 4 % by weight, preferably between 1 % and 3 % by weight; and/or the hyaluronic acid or one of its salts is present in an amount of between 0.3 % and 5 % by weight relative to the total weight of the composition, preferably between 0.5 % and 3 % by weight, preferably between 0.8 % and 2 % by weight.

9. Composition according to one of the preceding claims, **characterized in that** it comprises:
- an extracellular hemoglobin of *Arenicola marina* or of *Nereis virens,* preferably in an amount of between 0.05 % and 0.5 % by weight relative to the total weight of the composition, preferably between 0.08 % and 0.4 % by weight, preferably between 0.09 % and 0.3 % by weight;
- xanthan gum, preferably in an amount of between 0.5 % and 5 % by weight relative to the total weight of the composition, preferably between 1 % and 3 % by weight, preferably between 1.5 % and 2.5 % by weight; and
- sodium hyaluronate, preferably of high molecular weight, preferably in an amount of between 0.4 % and 5 % by weight relative to the total weight of the composition, preferably between 0.5 % and 4 % by weight, preferably between 0.8 % and 2 % by weight.

10. Composition according to one of the preceding claims, **characterized in that** it comprises:
- an extracellular hemoglobin of *Arenicola marina,* in an amount of between 0.09 % and 0.3 % by weight;
- xanthan gum in an amount of between 1.5 % and 2.5 % by weight; and
- sodium hyaluronate in an amount of between 0.8 % and 2 % by weight.

11. Device comprising:
- a syringe; and
- a composition according to one of the preceding claims.

12. Composition according to one of claims 1 to 10, for use as a drug.

13. Composition according to one of claims 1 to 10 for use for preventing and/or treating a periodontal disease, and/or for treating at least one periodontal pocket, and/or for promoting healing of the skin and/or of the periodontium, and/or for treating bone healing, and/or for preventing and/or treating a dermatological disease, preferably acne.
